# EUROPEAN PATENT APPLICATION

(11) **EP 1 538 201 A1**
(43) Date of publication of application: **08.06.2005**
(21) Application number: 04028416.8
(22) Date of filing: 01.12.2004
(51) Int. Cl.: C12N 9/12, C12P 21/02

(54) **Method for the recombinant production and purification of protein kinases**

(30) Priority: 02.12.2003 EP 03027755
(71) Applicant: F.HOFFMANN-LA ROCHE AG, 4070 Basel (CH)
(72) Inventor: Hertenberger, Hubert, 82362 Weilheim (DE); Honold, Konrad, 82377 Penzberg (DE); Klein, Christian, 82393 Iffeldorf (DE); Rueger, Petra, 82377 Penzberg (DE)
(74) Representative: Schreiner, Siegfried, Dr.

(57) **Abstract**

A method for the recombinant production and purification of a protein kinase selected from the group consisting of tyrosine protein kinases and serine/threonine kinases by expressing a nucleic acid encoding said kinase in a microbial host cell, forming inclusion bodies containing said kinase, isolating, solubilizing, naturing, and purifying said kinase characterized in that said purification is performed by hydrophobic interaction with an hydrophobic adsorbent under conditions whereby at least 70% of said correctly folded protein kinase are not bound to said adsorbent and the protein kinase not bound to said adsorbent is recovered.

## Description

The invention relates to an improved method for the recombinant production and purification of protein kinases in prokaryotes via inclusion bodies.

Protein kinases regulate many different cell proliferation, differentiation, and signaling processes by adding phosphate groups to proteins (Hunter, T., Cell 50 (1987) 823-829). Protein kinases are usually named after their substrate, their regulatory molecules, or some aspect of a mutant phenotype. With regard to substrates, the protein kinases may be divided into two groups; those that phosphorylate tyrosine residues (protein tyrosine kinases, PTK) and those that phosphorylate serine or threonine residues (serine/threonine kinases, STK). Almost all kinases contain a similar 250-300 amino acid catalytic domain. The N-terminal domain binds and orients the ATP (or GTP) donor molecule. The larger C terminal part binds the protein substrate and carries out the transfer of phosphate from ATP to the hydroxyl group of a serine, threonine, or tyrosine residue.

The kinases may be categorized into families by the different amino acid sequences (generally between 5 and 100 residues) located on either side of, or inserted into loops of, the kinase domain. These added amino acid sequences allow the regulation of each kinase as it recognizes and interacts with its target protein. The primary structure of the kinase domains is conserved and can be further subdivided into 11 subdomains. Each of the 11 subdomains contains specific residues and motifs or patterns of amino acids that are characteristic of that subdomain and are highly conserved (Hardie, G., and Hanks, S., The Protein Kinase Facts Books I, Academic Press, San Diego, Calif., 1995, pp. 7- 20).

The second messenger dependent protein kinases primarily mediate the effects of second messengers such as cyclic AMP (cAMP) cyclic GMP, inositol triphosphate, phosphatidylinositol 3,4,5-triphosphate, cyclic ADP ribose, arachidonic acid and diacylglycerol. Cyclic AMP dependent protein kinases (PKA) and mitogen-activated protein kinases (MAPK) are e.g. members of the STK family. Cyclic AMP is an intracellular mediator of hormone action in all procaryotic and animal cells that have been studied. Such hormone-induced cellular responses include thyroid hormone secretion, cortisol secretion, progesterone secretion, glycogen breakdown, bone resorption, and regulation of heart rate and force of heart muscle contraction. PKA is found in all animal cells and is thought to account for the effects of cyclic AMP in most of these cells. Altered PKA expression is implicated in a variety of disorders and diseases including cancer, thyroid disorders, diabetes, atherosclerosis, and cardiovascular disease.

MAP kinases like p38 also regulate intracellular signaling pathways. They mediate signal transduction from the cell surface to the nucleus via phosphorylation cascades. Several subgroups have been identified, and each manifests different substrate specificities and responds to distinct extracellular stimuli (Egan, S. E., and Weinberg, R. A., Nature 365 (1993) 781-783).

Protein kinase B (PKB/Akt) is a component of an intracellular signaling pathway of fundamental importance that functions to exert the effects of growth and survival factors, and which mediates the response to insulin and inflammatory signals (Datta, S.R., et al., Genes Dev. 13 (1999) 2905-2927; Brazil, D.P., and Hemmings, B.A., Trends Biochem. Sci. 11 (2001) 657-664). The recombinant production and PKB purification is described in WO 2003/016516 using Phenyl TSK hydrophobic interaction chromatography. PKB was adsorbed to the column and eluted after washing using a linear gradient.

Src kinases are implicated in cancer, immune system dysfunction and bone remodeling diseases. For general reviews, see Thomas, S.M., and Brugge, J.S., Annu. Rev. Cell Dev. Biol. 13 (1997) 513-609. Members of the Src family are e.g. Src, Fyn, Yes, Fgr, Lyn, Hck, Lck, and Blk. These are nonreceptor protein kinases that range in molecular mass from 52 to 62 kD. All are characterized by a common structural organization that is comprised of six distinct functional domains: Src homology domain 4 (SH4), a unique domain, SH3 domain, SH2 domain, a catalytic domain (SH1), and a C-terminal 15 regulatory region.

In prokaryotic organisms, the protein synthesis, also referred to as translation, takes place on the ribosomes in the cytoplasm. In expressing recombinant DNA in prokaryotic host organisms, such as, e.g., E. coli the resultant recombinant gene product/protein often precipitates in the cytoplasm in the form of insoluble inclusion bodies. After completion of fermentation and lysis of the cells, the inclusion bodies are isolated and optionally purified and the recombinant protein contained therein is solubilized by adding denaturants such as urea or guanidinium hydrochloride and naturation of said protein is accomplished by reducing the denaturing conditions. Such methods are well-known and have long been used successfully also for the industrial manufacture of recombinant proteins (cf., e.g., Lee, S.Y., Trends Biotechnol. 14 (1996) 98-105; Panda, A.K., et al., J. Biotechnol. 75 (1999) 161-172; Mattes, R., Semin. Thromb. Hemost. 27 (2001) 325-336; Clark, E.D., Curr. Opin. Biotechnol. 12 (2001) 202-207; Misawa, S., and Kumagai, I., Biopolymers 51 (1999) 297-307; and Lilie, H., Current Opinion Biotechnol. 9 (1998) 497-501).

However, expression of mammalian proteins in microbial host cells like E. coli is often a challenging task due to poor solubility, improper folding, lack of stability and other problems. Due to this the known procedures for the production and purification of protein kinases are very labor-intensive. Attempts of the inventors to produce protein kinases by recombinant expression in microbial host cells result in general only low amounts of active soluble kinases but with large amounts of undesired and inactive dimers and higher aggregates.

### Summary of the Invention

Now it was surprisingly found, that using the method of the invention kinases can be recovered after recombinant production in microbial host cells in a correctly folded form in large amounts with a very simple and robust method.

The object of the invention therefore is a method for the recombinant production and purification of a protein kinase selected from the group consisting of tyrosine protein kinases and serine/threonine kinases comprising
a) expressing a nucleic acid encoding said kinase in a microbial host cell,
b) forming inclusion bodies containing said protein kinase, and
c) isolating, solubilizing, naturing, and purifying said protein kinase,
characterized in that said purification is performed by hydrophobic interaction with an hydrophobic adsorbent under conditions whereby at least 70% of said correctly folded protein kinase are not bound to said adsorbent and the protein kinase not bound to said adsorbent is recovered. Unfolded protein is bound to the adsorbent.

Preferably said protein kinase is Src, PKB, c-Met, Lck, Aurora or p38 MAPK.

The adsorbent according to the invention is preferably a solid or gel material comprising cellulose, cross-linked dextran, cross-linked agarose or the like modified with hydrophobic residues like phenyl-, butyl-, or octylresidues (HIC adsorbent, hydrophobic interaction chromatography adsorbent).

Preferably the kinase is treated with the hydrophobic adsorbent in an aqueous solution containing at least 0.1M KCl or NaCl, more preferred 0.1 - 1M KCl or NaCl. Higher KCl or NaCl concentrations are possible as long as the protein kinase does not bind in an undesired large amount exceeding 70% of the total amount of protein kinase protein material. In addition high salt concentrations may destabilize protein kinases.

In addition the above hydrophobic interaction treatment is performed preferably in the presence of at least 0.5M arginine, guanidine or a compound having the general formula I

R₂-CO-NRR₁ (I),

or combinations thereof,
wherein
R and R₁ are hydrogen or a saturated or unsaturated branched or unbranched C₁-C₄ alkyl chain and
R₂ is hydrogen, NHR₁ or a saturated or unsaturated branched or unbranched C₁-C₃ alkyl chain.

### Detailed Description of the Invention

The invention relates to an improved method for the recombinant production and purification of protein kinases in prokaryotes via inclusion bodies.

The invention comprises a method for the recombinant production and purification of a protein kinase selected from the group consisting of tyrosine protein kinases and serine/threonine kinases by expressing a nucleic acid encoding said kinase in a microbial host cell, forming inclusion bodies containing said protein kinase, isolating, solubilizing, naturing, and purifying said protein kinase characterized in that said purification is performed by hydrophobic interaction with an hydrophobic adsorbent under conditions whereby at least 70% of said protein kinase are not bound to said adsorbent and the protein kinase not bound to said adsorbent is recovered.

The protein kinases which can be produced and purified according to the invention have been defined above. Preferably the method according to the invention is useful for the production and purification of Src kinases and cyclic AMP dependent protein kinases (PKA). Members of the Src family are e.g. Src, Fyn, Yes, Fgr, Lyn, Hck, Lck, and Blk. These are nonreceptor protein kinases that range in molecular mass from 52 to 62 kD. All are characterized by a common structural organization that is comprised of six distinct functional domains: Src homology domain 4 (SH4), a unique domain, SH3 domain, SH2 domain, a catalytic domain (SH1), and a C- terminal 15 regulatory region. Cyclic AMP dependent protein kinases (PKA) are members of the STK family. Cyclic AMP is an intracellular mediator of hormone action in all procaryotic and animal cells that have been studied. Such hormone-induced cellular responses include thyroid hormone secretion, cortisol secretion, progesterone secretion, glycogen breakdown, bone resorption, and regulation of heart rate and force of heart muscle contraction. PKA is found in all animal cells and is thought to account for the effects of cyclic AMP in most of these cells.

The above described protein kinases that can be produced according to the current invention, all play an important role in biochemical processes. For further elucidation of metabolic correlations not only the natural protein kinases are of interest but also mutants of these naturally occurring protein kinases.

An adsorbent (HIC or hydrophobic adsorbent) useful according to the invention comprises preferably a gel matrix substituted with hydrophobic ligands. The degree of substitution is usually in the range of 10-50µmol/ml gel. Preferred ligands are e.g. C2-C8 alkyl residues or simple aryl (like phenyl) residues. Usually hydrophobic interaction is increased by adding salt. If HIC adsorbents are used for chromatography the kinase in the correctly folded form is not bound to a considerable extent and therefore recovered in the flow-through.

Especially preferred is the use of a cross-linked agarose substituted with phenyl-, butyl- or octylgroups. Such adsorbent is for example phenyl-, butyl- or octyl-Sepharose (e.g. cross-linked agarose, 4% spherical, mean particle size 90µm, particle size range 45-165µm, degree of substitution approx. 50µmol butyl groups/ml gel), available from Amersham Biosciences (General Electric Healthcare).

The treatment with the hydrophobic adsorbent can be performed according to methods known to one of ordinary skill in the art, for example, treatment of the buffered solution containing the kinase with a buffered suspension of the adsorbent or as a chromatography (HIC).

The term "at least 70% of said protein kinase are not bound" means that from the protein recovered after recombinant production and naturation, which contains said protein kinase in a correctly folded form, not correctly folded form (e.g. multimers and the like) and other protein impurities from the host cell present after naturation in the aqueous solution containing the kinase, at least 70% of the correctly folded form are not bound to the adsorbent and found either in the supernatant after treatment of the kinase solution with the adsorbent or if the adsorbent is used within a chromatographic HIC purification found in the flow-through.

The term "correctly folded" means that the protein after naturation adopts the natural three dimensional structure. This is independent of catalytical activity and comprises also catalytically inactive mutants. Preferably active protein kinases are produced according to the current invention.

Binding of a correctly folded kinase to the adsorbent with a rate below 30% can be reached by treating an aqueous solution of such a kinase after naturation with at least 0.1M KCl or NaCl, more preferred 0.1 - 1M KCl or NaCl. Higher KCl or NaCl concentrations are possible as long as the protein kinase do not bind in an undesired large amount exceeding 70% of the total amount of protein kinase protein material. In addition very high salt concentrations may destabilize protein kinases.

As compounds having the general formula I there are preferably employed formamide, acetamide, urea or urea derivatives such as ethyl urea or methyl urea and salts thereof. Arginine, guanidine and salts thereof can be used, for example, as a hydrochloride or as another titrated form of the base. Preferably however L-arginine, more preferably the hydrochloride form of L-arginine, is employed.

Insoluble inclusion bodies are formed during recombinant expression of polypeptides in microbial host cells. Inclusion bodies are refractile aggregates of protease-resistant misfolded desired protein that occur upon over-expression of the encoding gene (Misawa, S., and Kumagai, I., Biopolymers 51 (1999) 297-307).

Suitable prokaryotic host cells for recombinant gene expression are, for example, gram-negative or gram-positive organisms, such as, e.g., E.coli and Bacillus subtilis. Suitable E.coli strains are, for instance, E.coli strains such as UT5600, AB101, XL1, K12, X1776 and W3110. However, other enterobacteriaceae as well as microorganisms such as Klebsiella, Salmonella or Bacillus subtilis, Pseudomonas or Streptomyces are also suitable as host cells. Also suitable as host cells are yeast strains, such as, e.g., Saccharomyces, Pichia, Hansenula, Kluyveromyces and Schizosaccharomyces.

The nucleic acid coding for the polypeptide is usually inserted in an expression vector. Suitable vectors are well-known to one skilled in the art and are, for example, plasmids or phages (Sambrook, J., Russell, D. W., Molecular Cloning, 3rd Edition, Chapter 15).

The fermentation of the host cells is also accomplished according to methods known to one skilled in the art. After a predetermined number of cells has been reached (measured via the optical density of the fermentation broth / cell suspension), the expression of the recombinant polypeptide is induced and cultivation is performed until the stationary phase is reached (in the case of batch cultures). After completion of cell growth, the cells are harvested and the inclusion bodies are isolated and processed by solubilization and naturation according to known methods.

After naturation not all protein kinase molecules are correctly folded, i.e. adopt the natural three dimensional structure. In fact a mixture of correctly folded molecules, not correctly folded molecules, aggregates and so on, is obtained. The separation and purification of correctly folded protein kinases from this mixture can be accomplished very easily by hydrophobic interaction with a hydrophobic adsorbent. With this method substantial amounts of correctly folded protein kinase can be purified to 95% purity and more.

The treatment with the hydrophobic adsorbent can be performed in the usual ways, e.g. treatment of the solution containing the kinase with a suspension of the adsorbent or as a chromatography (HIC), under conditions whereby at least 70% of said correctly folded protein kinase are not bound to said adsorbent and the protein kinase not bound to said adsorbent is recovered. Not correctly folded protein, dimers, etc. are bound to the adsorbent and thus removed from the solution.

If the adsorbent was added as e.g. suspension to the solution containing the protein kinase, the adsorbent is removed after the treatment by e.g. filtration with, e.g., a plaited paper filter.

The kinase recovered after the treatment with the hydrophobic adsorbent is still in solution and can be further processed depending on the estimated use according to methods known to a person skilled in the art, e.g. affinity chromatography, size-exclusion-chromatography, etc.

The following examples, figures and references are provided to aid the understanding of the present invention, the true scope of which is set forth in the appended claims. It is understood that modifications can be made in the procedures set forth without departing from the spirit of the invention.

### Description of the Figures

- **Fig. 1:**: Src activity after renaturation and hydrophobic chromatography as described. Activity is found in supernatant (SN) (EL=eluate).
- **Fig. 2:**: SDS page of renaturation and butyl chromatography fractions, Coomassie stained. While retaining activity in butyl supernatant (SN) high amounts of inactive Src protein are cut off. 1: Molecular weight protein standard, 2: Renaturation, 3: Renaturation+KCl, 4: Butyl SN, 5: Ultrafiltration/concentration.
- **Fig. 3:**: Activity of Src kinase after renaturation and addition of KCl or ammonium sulfate. Addition of ammonium sulfate that is common in hydrophobic chromatography results in loss of active Src in supernatant. KCl up to 1M keeps active Src in solution and allows separation of inactive Src on Butyl sepharose.
- **Fig. 4:**: SDS page, Coomassie Blue staining. Lane 1 standard proteins, lane 2 Aurora kinase preparation after refolding and Butyl Sepharose. Arrow: Aurora kinase.

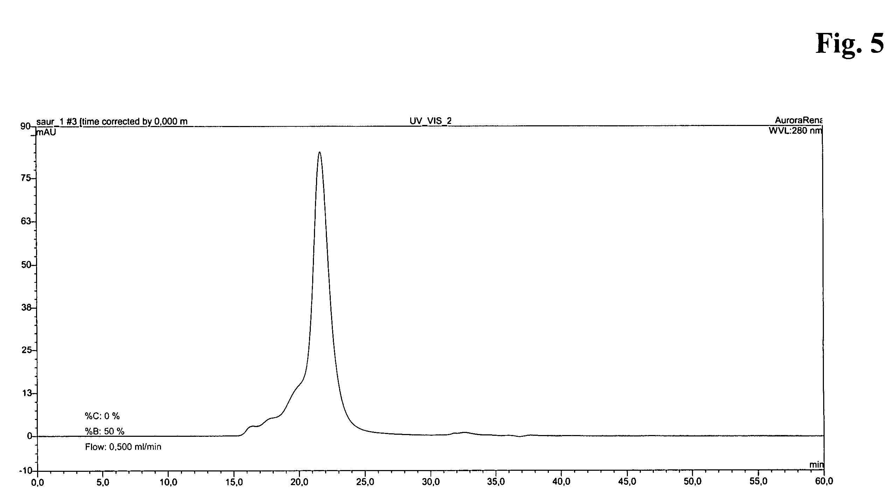

- **Fig. 5:**: Size exclusion chromatography of Aurora kinase after refolding and Butylsepharose batch. Superdex 75 (Pharmacia) 10/30. Buffer: 50mM TRIS pH7.5, 500mM NaCl, 10% Glycerol, 3mM CHAPS. Flow rate: 0.5ml/min.

### Example 1

### Recombinant production of Src

### a) Cloning

Constructs for the bacterial expression of mouse full length wild type Src kinase and derivatives for application in kinase assays and for structural purposes were cloned according to the following example procedure.

The ORF coding for mouse full length Src kinase was amplified from cDNA plasmid pUSE src wt (Upstate) via standard PCR using gene-specific oligonucleotide primers and Tgo/Tag DNA Polymerases (Roche Diagnostics GmbH), and subsequently sub cloned via BamHI and HindIII restriction sites into bacterial expression vector pQE32 (Qiagen GmbH). An N-terminal thrombin cleavage site was introduced by PCR. This vector served as a template for the generation of truncated and mutated versions of Src kinase domain and full length via site-directed mutagenesis. All inserts under control of the T5 promoter were confirmed by sequencing.

### b) Fermentation

Vectors plus co-vector pUBS520 (Brinkmann, U., Mattes, R. E., Buckel, P., Gene., 85 (1989) 109-114) were subsequently transformed into E. coli BL21 strain (Stratagene) for protein expression by large scale fermentation as inclusion bodies.

Proteins were expressed in E. coli BL21 grown at 37°C in LB medium supplemented with ampicillin (100 µg/ml) and kanamycin (50 µg/ml) to an absorbance of 0.5 -0.8 before overnight induction at 37°C with 0.5 mM IPTG (isopropyl-D-thiogalactopyranoside). After incubation, cells were harvested by centrifugation, resuspended and inclusion bodies were prepared according to the given procedure (Example 1, part c).

To prepare the inoculum 1 ml of a glycerol stock of the appropriate E. coli strain, harboring the expression plasmid to produce recombinant kinase protein, is added to 100 ml LB media and incubated for 8 to 10 hours on a rotary shaker at 37°C. This pre-culture is transferred to the sterilized fermenter vessel containing further LB media and glucose. The temperature of the main culture is maintained at 37°C when insoluble expression of the kinase protein to inclusion bodies is desired. The dissolved oxygen concentration of the media throughout the fermentation is kept above 20% saturation by increasing the agitation speed. Additional feeding of the culture is performed with the addition of glucose at rising pH values and the continuous dosing of a yeast-tryptone solution (2 ml/min). The fed batch fermentation ends when no more increase of the optical density is measurable. The culture broth is harvested by centrifugation.

### c) IB preparation

The biomass is suspended in a buffer containing TRIS and MgSO₄. Lysozym and DNase (e.g. Benzonase from Merck) are added if required. The bacteria cells are disrupted by homogenization to release the inclusion bodies from inside. Additional DNase keeps the suspension liquid what is essential for further handling. After an incubation period at room temperature a second buffer solution containing NaCl, EDTA and Brij solution is added to the suspension. The covered inclusion bodies are separated from the supernatant by centrifugation after an additional incubation time at room temperature. Then the pellet is suspended with a third buffer solution containing TRIS and EDTA to wash the IBs (endotoxin release) and incubated under stirring at room temperature and centrifuged.

### d) Naturation of Src

1.3g inclusion bodies were suspended in 100ml 0.1M TRIS pH 8.0, 8M Guanidine HCl, 10mM EDTA, 10mM DTT at room temperature. This Src solubilisate is added drop wise under stirring into 101 refolding buffer containing 1M TRIS pH 7.0, 0.5M Arginine, 10mM DTT, 10 tablets of Complete. The refolding process is continued 3-5 days at 8°C without stirring.

Dialysis against different buffers commonly used in the prior art (acetate, phosphate, MES, TRIS pH 5-9 with various additives) at this stage was not successful due to high aggregation of inactive, not correctly folded Src and then co-immunoprecipitation of the active fraction. It was necessary to remove the not correctly folded, inactive Src solubilized in renaturation buffer by hydrophobic chromatography before dialysis.

### e) Hydrophobic batch chromatography

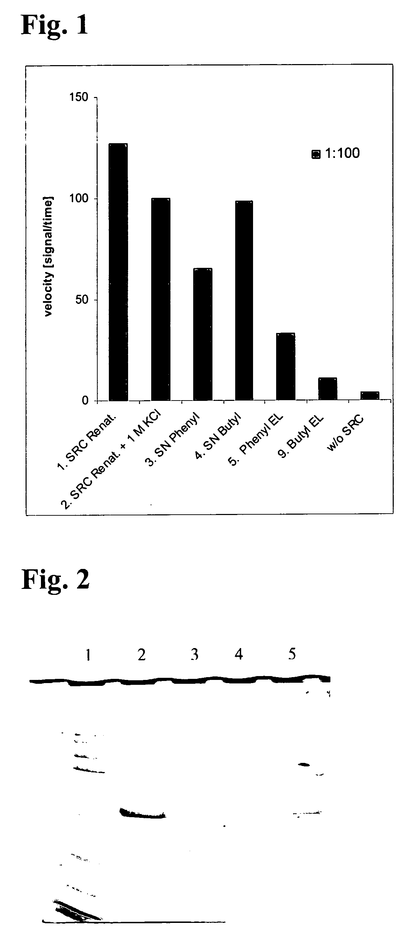

The refolded protein solution was set to 1M KCl. Subsequently 40g Butylsepharose 4 Fast Flow (Amersham Biosciences) were added and binding allowed for 1h at 8°C. After removal of Butylsepharose by filtration the supernatant contains the correctly folded, active Src protein. Under these conditions not correctly folded, non-active protein is bound to Butylsepharose. This method allows separation of active and inactive Src (Fig. 1 and 2). Further purification can be achieved if necessary by additional chromatography steps, e.g. Ni-chelate chromatography, size exclusion chromatography, etc.

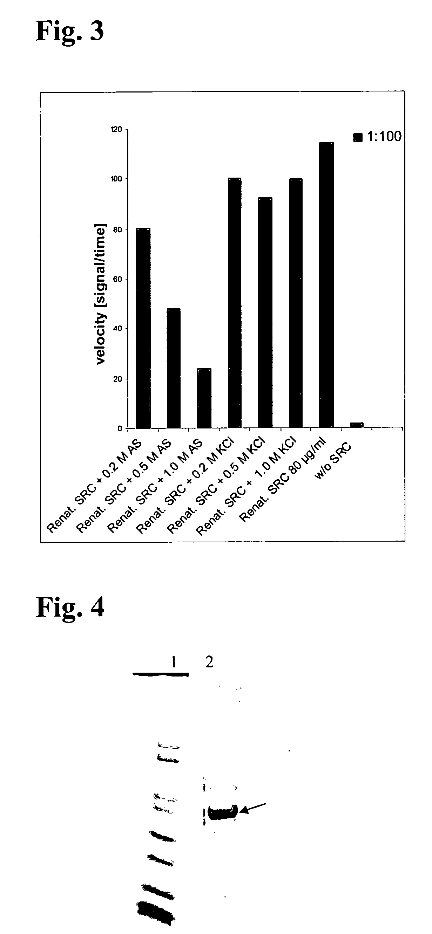

Addition of KCl (0.2M, 0.5M and 1.0M) proved to be superior versus the prior used ammonium sulfate as can be seen in Fig. 3. Similar concentrations of ammonium sulfate already result in precipitation of active Src. Using conditions that lead to binding of the active Src on hydrophobic materials were not suitable because of high loss of activity on the column. Eluates show only low Src protein and activity recovery.

### f) Src assay

Phosphorylation of Src substrate peptide YA133 labeled by Src kinase is measured by using a phosphotyrosine antibody Eu labeled (PT66 Lance Eu-W1024 (Wallac)) and detection of time resolved fluorescence signal.

### Example 2

### Recombinant production of Aurora A kinase

### a) Cloning

Vector constructs for the bacterial expression of human full-length wild type Aurora A kinase and derivatives for application in kinase assays (e.g. Elisa, HTRF, FP) and for biostructural purposes were designed and cloned according to the following procedure.

The ORF coding for human full-length Aurora A kinase (residues 1-403) was amplified from a human HeLa cDNA library (Clontech) via standard PCR using gene-specific oligonucleotide primers and Pwo DNA polymerase (Roche Diagnostics GmbH), and subsequently sub cloned into bacterial expression vectors. These vectors served as a template for the generation of truncated versions of Aurora A via PCR using gene-specific oligonucleotide primers and of Aurora A muteins via site-directed mutagenesis. For the final expression constructs wild type and mutant Aurora A kinase domains (residues 114-403) were amplified from the corresponding Aurora A basic vectors via PCR using gene-specific primers and Pwo DNA polymerase. PCR products were sub cloned via Ndel and XhoI restriction sites into modified pQE40 expression vectors under control of a T5 promoter with and without an N-terminal RGS-(His)₆-tag. All vector insert sequences were confirmed by sequencing. Vectors were subsequently transformed into E. coli BL21, 20 and UT5600 strains co-transformed with the pUBS520 co-repressor plasmid (Brinkmann, U., Mattes, R. E., Buckel, P., Gene. 85 (1989) 109-114). Aurora protein was subsequently expressed by large scale fermentation as inclusion bodies. Proteins were expressed in E. coli strains BL21 and UT5600 grown at 37 °C in LB medium supplemented with ampicillin (100 µg/ml) and kanamycin (50 µg/ml) to an absorbance of 0.5-0.8 before overnight induction at 37°C with 1 mM isopropyl-D-thiogalactopyranoside. After induction, cells were harvested by centrifugation, resuspended and inclusion bodies were prepared according to the given procedure.

### b) Fermentation

To prepare the inoculum 1 ml of a glycerol stock of the appropriate E. coli strain, harboring the expression plasmid to produce recombinant kinase protein, is added to 100 ml LB media and incubated for 8 to 10 hours on a rotary shaker at 37°C.

This pre-culture is transferred to the sterilized fermenter vessel containing further LB media and glucose. The temperature of the main culture is maintained at 37°C. Additional feeding of the culture is performed with the addition of glucose at rising pH values and the continuous dosing of a yeast-tryptone solution (2 ml/min). The fed batch fermentation ends when no more increase of the optical density is measurable. The culture broth is harvested by centrifugation.

### c) IB preparation

The biomass is suspended in a buffer containing TRIS and MgSO₄. Lysozym and DNase (e.g. Benzonase from Merck) are added if required. The bacteria cells are disrupted by homogenization to release the inclusion bodies from inside. After an incubation period at room temperature a second buffer solution containing NaCl, EDTA and Brij solution is added to the suspension. The covered inclusion bodies are separated from the supernatant by centrifugation after an additional incubation time at room temperature. Then the pellet is suspended with a third buffer solution containing TRIS and EDTA to wash the IBs and incubated under stirring at room temperature and centrifuged.

### d) Naturation of Aurora A

160mg inclusion bodies were suspended in 10ml 0.1M TRIS pH 8.0, 8M Guanidin HCl, 10mM EDTA, 10mM DTT at room temperature. This Aurora A solubilisate is added drop wise under stirring into 11 refolding buffer containing 1M TRIS pH 7.0, 0.5M Arginine, 10mM DTT. The refolding process is continued 1 day at 8°C without stirring.

### e) Hydrophobic batch chromatography

The refolded protein solution was set to 1M KCl. After 30 min 5g Butylsepharose 4 Fast Flow (Amersham Biosciences) were added and binding allowed for 1h at 8°C. After removal of Butylsepharose by filtration the supernatant contains mainly the correctly folded, active Aurora A protein. Under these conditions not correctly folded protein is bound to Butylsepharose. Further purification can be achieved if necessary by additional chromatography steps, e.g. ion exchange, size exclusion chromatography, etc.

### f) Analysis of refolded protein

After refolding the supernatant of the Butyl Sepharose batch contains >90% monomeric Aurora A kinase as is seen by SDS-Page and size exclusion chromatography.

### List of References

Brazil, D.P., and Hemmings, B.A., Trends Biochem. Sci. 11 (2001) 657-664
Brinkmann, U., Mattes, R. E., and Buckel, P., Gene 85 (1989) 109-114
Clark, E.D., Curr. Opin. Biotechnol. 12 (2001) 202-207
Datta, S.R., et al., Genes Dev. 13 (1999) 2905-2927
Egan, S. E., and Weinberg, R. A., Nature 365 (1993) 781-783
Hardie, G., and Hanks, S., The Protein Kinase Facts Books I, Academic Press, San Diego, Calif., 1995, pp. 7- 20
Hunter, T., Cell 50 (1987) 823-829
Lee, S.Y., Trends Biotechnol. 14 (1996) 98-105
Lilie, H., Current Opinion Biotechnol. 9 (1998) 497-501
Mattes, R., Semin. Thromb. Hemost. 27 (2001) 325-336
Misawa, S., and Kumagai, I., Biopolymers 51 (1999) 297-307
Panda, A.K., et al., J. Biotechnol. 75 (1999) 161-172
Sambrook et al., ed., Molecular Cloning: A Laboratory Manual, 2^{nd} ed., Cold Spring Harbor Laboratory Press, Cold Spring Harbor, NY, 1989, Chapter 15
Thomas, S.M., and Brugge, J.S., Annu. Rev. Cell Dev. Biol. 13 (1997) 513-609 WO 2003/016516

## Claims

1. Method for the recombinant production and purification of a protein kinase selected from the group consisting of tyrosine protein kinases and serine/threonine kinases comprising
a) expressing a nucleic acid encoding said kinase in a microbial host cell,
b) forming inclusion bodies containing said kinase, and
c) isolating, solubilizing, naturing, and purifying said kinase,
**characterized in that** said purification is performed by hydrophobic interaction with an hydrophobic adsorbent under conditions whereby at least 70% of said correctly folded protein kinase are not bound to said adsorbent and the protein kinase not bound to said adsorbent is recovered.

2. Method according to claim 1, **characterized in that** the kinase is Src, PKB, c-Met, Lck or p38 MAPK.

3. Method according to claim 1 or 2, **characterized in that** the adsorbent is phenyl-, octyl- or butyl-sepharose

4. Method according to claims 1 to 3, **characterized in that** the kinase is applied to the chromatographic material in an aqueous solution containing at least 0.1M KCl or NaCl.

5. Method according to claim 4, **characterized in that** the aqueous solution contains in addition at least 0.5M arginine, guanidine or a compound having the general formula I
R₂-CO-NRR₁ (I),
or combinations thereof,
wherein
R and R₁ are hydrogen or a saturated or unsaturated branched or unbranched C₁-C₄ alkyl chain and
R₂ is hydrogen, NHR₁ or a saturated or unsaturated branched or unbranched C₁-C₃ alkyl chain.
